Europäisches Patentamt

(19) European Patent Office

Office européen des brevets

(11) Publication number: 0 174 096
A2

## (12) EUROPEAN PATENT APPLICATION

(21) Application number: 85305529.1

(22) Date of filing: 02.08.85

(51) Int. Cl.⁴: **C 12 N 15/00**
**C 12 P 21/02**

(30) Priority: 02.08.84 US 637131
19.07.85 US 757014

(43) Date of publication of application:
12.03.86 Bulletin 86/11

(84) Designated Contracting States:
AT BE CH DE FR GB IT LI NL SE

(71) Applicant: BIOTECHNICA INTERNATIONAL, INC.
85 Bolton Street
Cambridge Massachusetts(US)

(72) Inventor: Backman, Keith C.
200 Carlisle Road
Bedford Massachusetts(US)

(74) Representative: Deans, Michael John Percy et al,
Lloyd Wise, Tregear & CO. Norman House 105-109
Strand
London WC2R OAE(GB)

(54) Vectors for introducing DNA into bacterial chromosomes or for deleting it therefrom and for the production of protein.

(57) An integration vector which may be used as a cloning vehicle for an engineered DNA sequence one wishes to integrate into a bacterial chromosome comprises a replication-specific sequence capable of directing autonomous replication and stable maintenance of the vector in a bacterial host cell, and two restriction endonuclease cleavage sites bracketing the replication specific sequence so that the replication specific sequence can be conveniently excised. Upon excision of the replication specific sequence, the engineered DNA is capable of integration into the chromosome of a bacterial host cell of the same type as the host cell used for autonomous replication and stable maintenance, thus enabling replication of the engineered sequence as part of the bacterial chromosome.

Precursors for such vectors are also described.

Methods using such vectors are described for the microbiological production of a desired protein, for introducing artificially engineered DNA into the chromosome of a bacterial cell to replace a segment of chromosomal DNA, and for deleting a specific chromosomally located gene from a bacterial cell, are also described.

./...

FIG 1a         FIG 1b

FIG 1d         FIG 1c

FIG 1e         FIG 1f

FIG 1h         FIG 1g

VECTORS, PRECURSORS THEREFOR, AND METHODS USING SUCH
VECTORS FOR INTRODUCING ARTIFICIALLY ENGINEERED DNA
INTO THE CHROMOSOME OF A BACTERIAL CELL, FOR DELETING
A SPECIFIC CHROMOSOMALLY LOCATED GENE FROM A BACTERIAL
CELL, OR FOR THE MICROBIOLOGICAL PRODUCTION OF PROTEIN

This invention relates to vectors, to precursors, and to methods using such vectors for introducing artificially engineered DNA into the chromosome of a bacterial cell, for deleting a specific chromosomally located gene from a bacterial cell, or for the microbiological production of protein.

In the construction of living organisms with genetic engineered traits, it is desirable to integrate engineered genes into the chromosome of a cell. Altered copies of genes located on extrachromosomal elements (plasmids) have a tendency to suffer mutations or be lost from the cell during growth, thereby reducing product yield. In contrast, chromosomal DNA tends to be faithfully preserved during replication, and the genetic modification of a chromosomally located gene is stably maintained in the progeny of the engineered cell.

Because the engineered DNA generally will not recombine into the chromosome of a host cell if the cloning vehicle is capable of autonomous replication in that cell, the ultimate host for the engineered gene should be one in which the vehicle is not capable of replication. Therefore, the method for alteration of a chromosomally located gene usually involves the use of two hosts. A cloning vehicle containing a genetically engineered gene is obtained in a host of a first cell type where it can replicate. Then it is transferred to a second cell-type host for integration into that cell's chromosome. Usually the DNA introduced into the second host is incapable of replication either by virtue of an inherent lack of an appropriate replication origin, or because the second host is incapable of causing the plasmid to replicate. One such situation involves a plasmid engineered in E. coli (a convenient, well-characterised host for plasmid engineering) and intended for integration into the chromosome of a different type of organism (e.g a yeast or a Bacillus subtilis) which cannot cause the E. coli plasmid to replicate. See, for example, Haldenwang et al. (1980) J. Bacteriology 142 (1): 90-98; Scherer et al. (1979) Proc. Nat'l. Acad. Sci. USA 76 (10): 4951-4955. Where it is desirable to integrate the engineered gene into the chromosome of the same type of cell as is used for the first host, other provisions must be made, e.g., mutations in the second host cell which render the plasmid DNA incapable of replication in that environment (e.g. Gutterson and Koshland, "Replacement and amplification of bacterial genes with sequences altered in vitro", Proc. Nat'l. Acad. Sci., Vol. 80, pp. 4894-4898, August 1983).

In accordance with a first aspect of the present invention, we provide a vector characterised in comprising: a replication-specific sequence, having a first cleavage site recognised by a restriction endonuclease adjacent one end of said replication specific sequence, and a second cleavage site recognised by a restriction endonuclease adjacent the other end of said replication specific-sequence, said first and said second sites being the only sites on said vector recognised by a restriction endonuclease which recognises either said first or said second cleavage site; an engineered DNA sequence to be integrated into the chromosome of a selected bacterial cell; and means allowing detection of bacterial cells that carry said engineered sequence; and further characterised in that said vector is capable of stable maintenance and autonomous replication of said engineered sequence in a bacterial cell; and in that, upon excision of said replication-specific sequence, said engineered sequence is effective to integrate into the chromosome of a bacterial cell of the same type as the cell in which said vector can be stably maintained, the engineered sequence so integrated then

being replicated as part of said chromosome.

It will be seen that the vector includes a replication specific sequence, i.e., a sequence capable of directing autonomous replication and stable maintenance of the vector in a bacterial host cell. The vector also includes two restriction endonuclease cleavage sites bracketing the replication specific sequence, so that the replication specific sequence may be conveniently excised. Upon excision of the replication specific sequence, the engineered DNA sequence is capable of integration into the chromosome of a bacterial host cell of the same type as the host cell used for autonomous replication and stable maintenance, thus enabling replication of the engineered sequence as part of the bacterial chromosome.

The invention extends to precursors to such vectors, the precursors lacking the engineered sequence, and having a cleavage site recognised by a restriction endonuclease that does not recognise either of the sites bracketing the replication-specific site.

We describe in more detail below methods by which an engineered DNA sequence can be replicated extra-chromosomally in one type of bacterial host cell and then can be integrated into the chromosome of a bacterial cell of the same type.

The invention accordingly provides, in a second and alternative aspect thereof, a method by which artificially engineered DNA can be introduced into the chromosome of a bacterial cell to replace a segment of chromosomal DNA, characterised in that it comprises the steps of

(a) maintaining said engineered DNA in a first bacterial cell on a vector comprising: the said engineered DNA, a replication-specific sequence, having a first cleavage site recognised by a restriction endonuclease adjacent one end of said replication-specific sequence, and a second cleavage site recognised by a restriction endonuclease adjacent the other end of said replication specific-sequence, said first and said second sites being the only cites on said vector recognised by a restriction
endonuclease which recognises either said first or said
second cleavage site, and means allowing detection of
bacterial cells that carry said engineered sequence; said
vector being capable of stable maintenance and autonomous
replication of said engineered sequence in said first
bacterial cell;

(b) exposing said vector to a said restriction endonuclease, and removing a DNA segment comprising said
replication-specific sequence;

(c) recircularising the remaining DNA;

(d) transforming a second bacterial cell of the same type
as said first cell with said recircularised remaining DNA
so that said engineered DNA is incorporated by recombination
into the chromosome of said cell and thereby replaces said
segment of chromosomal DNA; and

(e) identifying descendants of said transformed cells in
which the engineered DNA is integrated into the chromosome.

In preferred arrangements, the replication-specific
sequence is a replication origin derived from a plasmid;
for example, the host cell is E. coli and the replication-
specific sequence is the origin of replication of pBR322.
The DNA sequence which provides the means for detecting
bacterial cells carrying the engineered sequence is
preferably a sequence conferring antibiotic resistance.
Also in preferred

embodiments, at least some of the steps involved in engineering the DNA sequence take place on the vector while it is maintained in a host bacterial cell. The engineered DNA preferably includes flanking sequences at either end which are homologous to the flanking sequences at either end of a chromosomal DNA sequence to be replaced.

Also in preferred embodiments, the vector includes a selectable marker between the first and second restriction enzyme cleavage sites to mark the presence of the replication-specific sequence and thereby to differentiate integrants from cells having the replication-competent extrachromosomal plasmid. Either alone or in combination with the above-mentioned selectable marker, the replication specific sequence and other DNA to be excised may be positioned to divide a gene coding for a selectable marker into two non-functional fragments that are joined and become functional upon excision of that fragment. In this way, when the replication incompetent DNA integrates in the host chromosome, the second selectable marker will be expressed. Thus it is possible to select for cells that have undergone the desired integration by selecting for the second selectable marker. Most preferably, the segment of DNA to be integrated carries a marker that can also be selected against (like tetracycline resistance) and that is identical to the second selectable marker, so that after colonies exhibiting the trait are established, the trait can be selected against to isolate microorganisms that have undergone excision of the marker gene. By so-doing, the process of identifying the ultimately desired cells is simplified because that identification is made from a population containing a larger percentage of desired cells.

The method may be used specifically to integrate into the chromosome an engineered gene coding for a desired protein so that the second host produces the desired protein; alternatively, the method may be used to delete a specific chromosomally located gene from a bacterial cell by constructing a hybrid DNA molecule comprising in the correct orientation two discrete DNA sequences, one of which is naturally located immediately 5' to the gene to be deleted, and the other of which is naturally located 3' to the gene. The DNA molecule is inserted into a vector from which the replication origin can be conveniently excised. Upon excision of the replication origin from the engineered construction, the remaining DNA is recircularised, and the newly-made construction is incorporated by recombination into the chromosome of the bacterial cell at or near the locus of the gene for which the construction carries an engineered copy.

Accordingly, the invention provides, in a further alternative aspect thereof a method for deleting a specific chromosomally located gene from a bacterial cell, said method being characterised in comprising the steps of: ·
(a) forming an engineered DNA sequence by ligating together in the correct orientation two discrete DNA sequences, one of which is naturally located immediately 5' to the gene to be deleted, and the other ordinarily located 3' to the said gene to form a hybrid DNA molecule;
(b) inserting said hybrid DNA molecule into a vector from which a DNA sequence essential for replication can be conveniently excised;
(c) excising said replication sequence from the vector and recircularizing the remaining DNA;
(d) transforming a said bacterial cell with said recircularized remaining DNA, so that said hybrid DNA molecule is incorporated by recombination into the chromosome of said cell and thereby replaces said chromosomally located gene; and

(e)   identifying descendants of the said cells having said hybrid DNA molecule integrated into said cell's chromosome in place of said gene.

The alteration of a chromosomally located gene ensures that the alteration will be stably maintained in the progeny of the engineered cell.  The use of a vector which can be prepared and stably maintained in the same type of host cell that is ultimately to carry the chromosomally located alteration overcomes problems inherent in systems that involve the use of the two different types of hosts, such as restriction modification system barriers between heterologous cell types.  The vectors and methods described herein simplify the procedure for alteration of a chromosomally located gene and expand the applicability of such a procedure.

The invention is hereinafter more particularly described by way of example only with reference to the accompanying drawings, in which:-

Figs. 1a-1h are flow diagrams of the steps of a method for introducing engineered genes into the chromosomal DNA of a bacterial cell; the diagram indicating certain restriction endonuclease sites on the vector DNA and chromosomal DNA;

Fig. 2 is a diagram of the vector pKB701 showing certain restriction endonuclease sites thereon;

Fig. 3 is a diagram depicting the construction of pKB719;

Fig. 4 is a diagram depicting the construction of pKB843; and

Figs. 5a - 5h are flow diagrams of the steps of an alternate method for introducing engineered genes into the chromosomal DNA of a bacterial cell.

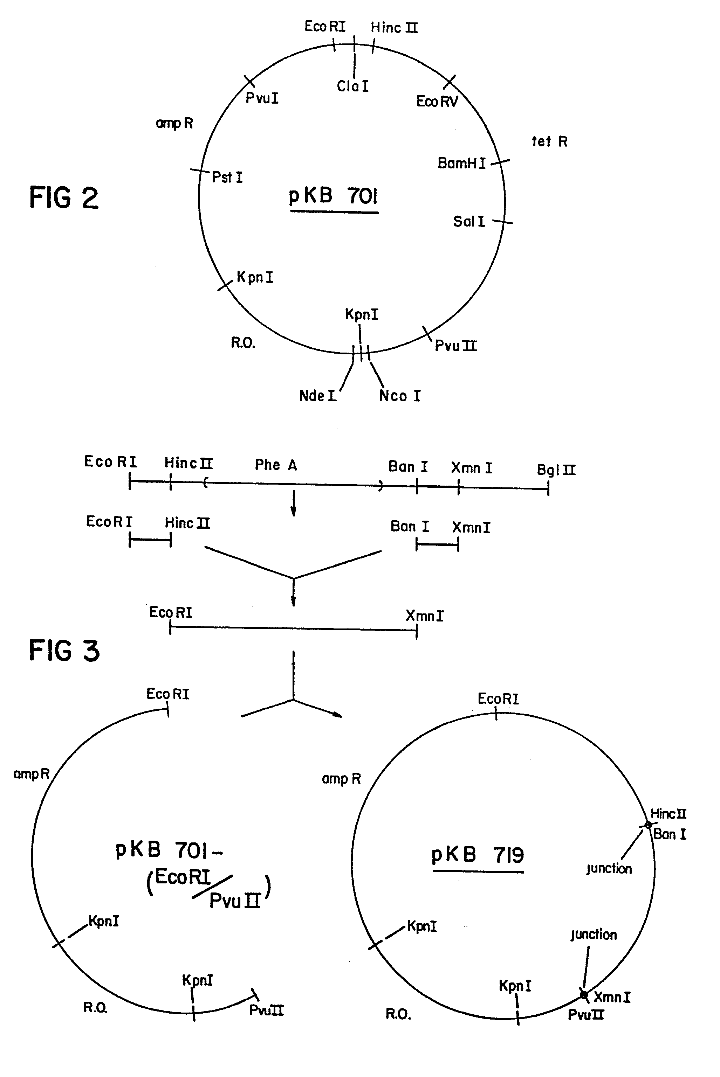

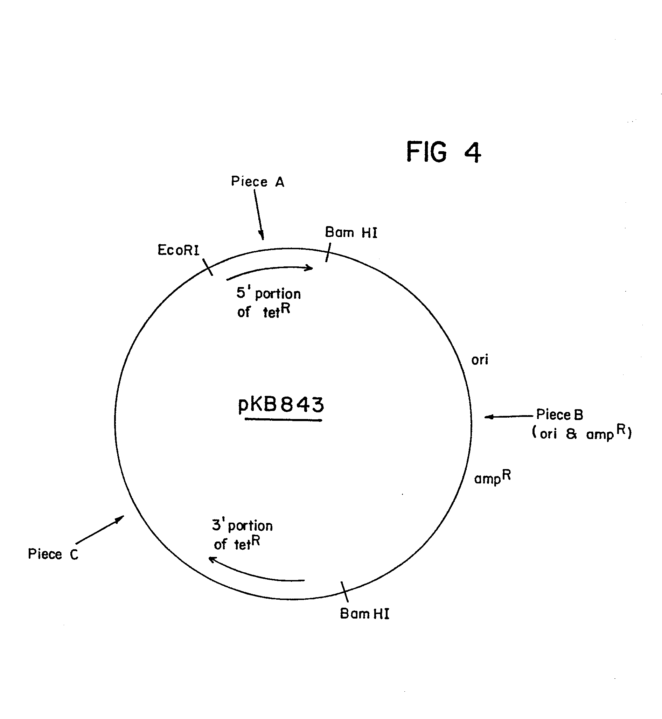

In general, the vector is constructed from a precursor (e.g. pKB701 in Fig. 2 or pKB843 illustrated in Fig. 4) which includes a replication origin bracketed on either side by restriction endonuclease sites. These two restriction endonuclease sites may be sites that are recognized by the same or different enzymes. Located outside of the replication origin sequence is a cleavage site for the insertion of the DNA sequence one wishes to engineer. In the preferred embodiment this restriction endonuclease cleavage site is recognized by an enzyme which does not recognize either of the first two sites. The vector further includes a DNA sequence, or sequence segments which, when joined, code for an antibiotic resistance trait which provides the means by which bacterial cells carrying the engineered sequence on a chromosome may be detected. The structure of two specific such precursors (pKB701 and pKB843) are illustrated by the following description of their construction.

The following description of the construction of vectors pKB701 and pKB843 illustrates methods for making the vectors; to the extent not specifically described herein, standard recombinant DNA techniques are used such as those described in Maniatis, _Molecular Cloning_ (Cold Spring Harbor Laboratories 1982) which is hereby incorporated by reference.

To construct the vector pKB701 (see Fig. 2), a derivative of pBR322 was constructed in which KpnI linkers were inserted into sites adjacent to and bracketing the replication origin. On the side of the replication origin adjacent the beta lactamase gene, this was accomplished by partial digestion with Sau 3A. Initially, a HindIII linker was put between the gene for beta-lactamase and the origin. Later, this Hind III site was converted to a KpnI site using KpnI linkers. Similarly, the Tth111I site of pBR322 was converted to a KpnI site using KpnI linkers. The specifics of the construction yielded an NcoI site (at the junction of the KpnI linker and the Tth111I site) and an NdeI site (between adjacent KpnI linkers which occur in the construction). The two linker insertions were combined onto a single plasmid in a construction which involved NdeI and EcoRI. As a consequence, a small amount of material between the NdeI and Tth111I sites of pBR322 was lost from the final vector. The original insertion of a Hind III linker was also accompanied by a small loss of material, but neither of these small deletions has a detectable consequence on the utility of the vector.

A second precursor vector pKB843 is constructed from the elements illustrated in Fig. 4 and includes certain improvements that make its use more efficient. The details of the elements of pKB843 are clear from the description of its construction given below. In brief, pKB843 contains two selectable markers that aid in selection of cells that have undergone the desired integration. When using pKB701, the origin of replication must be completely eliminated; otherwise it will not be possible to use the selectable marker to differentiate between colonies where integration has occurred and colonies where the marker is present on an extrachromosomal element. Plasmid pKB843 addresses this problem as described below.

Plasmid pKB843 contains a gene encoding a selectable phenotype on the segment that is deleted with the origin of replication. As shown in Fig. 4, pKB843, piece B contains the origin of replication from pBR322 and $amp^R$. Thus, the presence of ampicillin resistance indicates the presence of the extrachromosomal plasmid pKB843 with its origin of replication.

Plasmid pKB843 contains a gene coding for a second selectable phenotype (tetracycline resistance). The gene is divided by piece B into two inactive fragments. When the origin of replication is deleted and the resulting vector integrates, the two fragments will be joined to form a functional $tet^R$ gene that can be used to differentiate integrants from cells having plasmids that retain an origin of replication. As described below it is also useful to select against $tet^R$ to isolate cells that have undergone excision.

Plasmid pKB843 also has a suitable restriction endonuclease cleavage site for insertion of a sequence to be combined in the host chromosome; for example, EcoRI in Fig. 4.

11

To construct pKB843, three separate segments of DNA shown in Fig. 4 are obtained and ligated together using recombinant DNA engineering techniques such as those described by Maniatis, cited above.

Piece A is a segment containing the 5' portion of the tet$^R$ gene, such as the 375 bp fragment obtained by digesting pBR322 with EcoRI and BamHI.

Piece B is a 2.14 kb segment of pBR322 comprising the region from Tth111I to EcoRI and carries the origin of replication as well as an ampicillin resistance gene or a DNA segment having a similar function. The segment is obtained by digestion with EcoRI and Tth111I, and the ends of the segment are converted by linkers first to KpnI ends and then to BamHI ends.

Piece C is any suitable region having a BamHI site adjacent a 3' portion of the tet$^R$ gene that complements the 5' portion of piece A to yield a functional tet$^R$ gene. A suitable fragment of pBR322, or a precursor to it, could be used such as a BamHI-to-HpaI fragment of pMB9 [ATCC 37019] in which a linker has been used to convert the HpaI site to EcoRI.

Plasmids pKB701 and pKB843 have been deposited in E. coli strain YMC9 with the American Tissue Type Culture Collection under accession nos. 39772 and 53181, respectively.

In general, the precursor vector is utilized as follows: Referring to Figs. la and lb, a DNA sequence A is inserted into the precursor vector which has a replication origin RO and a marker M to convey antibiotic resistance and facilitate recognition of cells carrying the vector. The insertion of sequence A into the precursor is facilitated by the existence on the precursor of a cleavage site recognized by an enzyme which does not recognize either of the sites bracketing the replication origin. Sequence A may be any sequence that is to be engineered to sequence A' for integration into the chromosome--for example, it may be a structural gene coding for a protein desirable in its own right or an enzyme which catalyzes a reaction in a pathway to a desired product, together with an appropriate control sequence for expression of that structural gene. Sequence A includes flanking regions at each of its ends which are homologous to, and have the same direction as, corresponding regions of host chromosomal sequence $A_c$ which is targeted to be replaced by sequence A'. These flanking regions must be long enough to permit integration of sequence A' into the chromosome by means of recombination events. Specifically, the efficiency of introducing construction A' into the chromosome will increase as the size of the homologous flanking regions increases.

Referring to Fig. lc, the DNA sequence is engineered so that sequence A becomes altered sequence A' as a result of engineering of desired changes in sequence A.. In Fig. lc the vector containing the altered sequence A' can autonomously replicate in a host cell, and the vector will be stably maintained because of the presence of the replication origin RO on the vector. In all steps, the vector and gene under engineering may be maintained in the same cell type as is ultimately to carry the alteration on its chromosome.

To effect integration of the altered sequence into the chromosome of a host cell, the plasmid is isolated. The replication origin RO then is excised (Fig. 1d) by restriction endonuclease digestion and separated from the remainder of the DNA, which is recircularized by treatment with DNA ligase. The vector is incorporated into a host where it recombines into the cell chromosome at or near the locus of the sequence $A_c$ for which the vector carries an engineered copy A' (Fig. 1e). Only those constructions which have lost the replication origin will be incorporated into the cell chromosome. The proportion of altered (A') chromosomes to wild-type ($A_c$) chromosomes produced as a result of recombination will be greatest when the sizes of the flanking regions are generally equivalent to each other because this will favor equal rates of recombination on each side of the alteration.

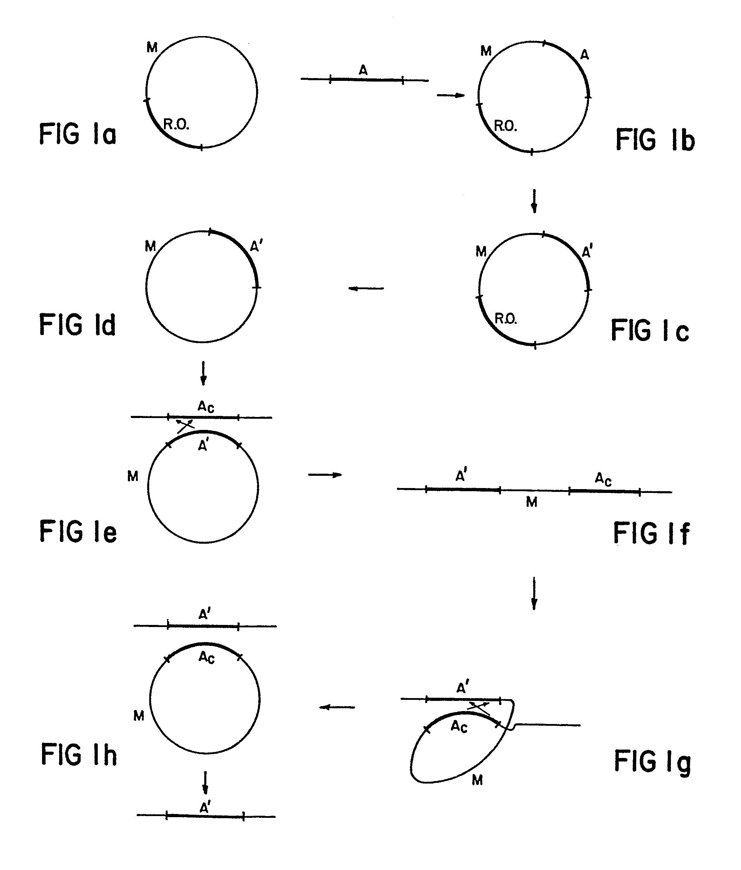

Referring to Fig. 1f, a cross-over recombination event occurs so that the altered sequence A' is carried on the cell chromosome and the naturally occurring sequence $A_c$ is carried on the vector (Fig. 1g).

The construction and use of a specific vector (pKB719) illustrates the use of the above procedure to delete a chromosomally located gene. Referring to Fig. 3, in order to have an E. coli strain in which all chromosomal sequences comprising the pheA structural gene were removed, a cloned version of the E. coli pheA gene was used. A piece of DNA was constructed which contained material from either side of the pheA gene.

14

On one side, the region EcoRI to Hinc II was used; on the other, the Ban I to XmnI region was used. These regions were combined and were cloned between the EcoRI and PvuII sites of pKB701, yielding pKB719. pKB719 was digested with KpnI, and the resulting fragments were resolved by polyacrylamide gel electrophoresis. The larger fragment, carrying the engineered pheA deletion, was circularized with DNA ligase and transformed into E. coli strain YMC9 (ATCC 33927). Ampicillin resistant transformants were selected. These occurred at a frequency between 100 and 1,000 times less than transformation of YMC9 with pBR322. Transformants were analyzed for the presence of plasmid DNA. If there is contamination of the prepared DNA fragment with either the origin fragment or incompletely digested pKB719, then some transformants will carry plasmids identical to pKB719. The frequency of recovery of pKB719 transformants may be sharply reduced by treating the circularized DNA with restriction endonuclease NdeI, which linearizes DNA molecules containing the replication origin fragment. Several ampicillin resistant isolates which did not carry any detectable plasmid were identified. Cultures of these isolates were enriched for phenylalanine auxotrophs by cycloserine enrichment. Such phenylalanine auxotrophs were found to have simultaneously lost their ampicillin resistance, indicating that they arose by a mechanism outlined in Fig. 1f.

The above-mentioned problems regarding the existence of transformants having plasmids identical to pKB719, or other plasmids containing the $amp^R$ selection marker on a replication competent extrachromosomal element, is ameliorated by the use of plasmids derived from pKB843 because of the improved mechanism for selecting organisms that have undergone

15

the desired events. After the desired chromosomal-insertion fragment has been inserted in pKB843, and the replication origin has been deleted as described above, the resulting plasmid is inserted in a host organism and tetracycline resistant transformants are selected. An amount of tetracycline that is appropriate for cells carrying a single copy of $tet^R$ is used, most preferably about 3 μg/ml.

The $amp^R$ marker is used to ensure that the selected transformants are characterized by an integrated $tet^R$ gene and not by a $tet^R$ gene on a replication competent plasmid such as may arise rarely by the joining of an origin fragment to two of the replicationally inert fragments, generating a replication competent plasmid bearing the $tet^R$ gene.

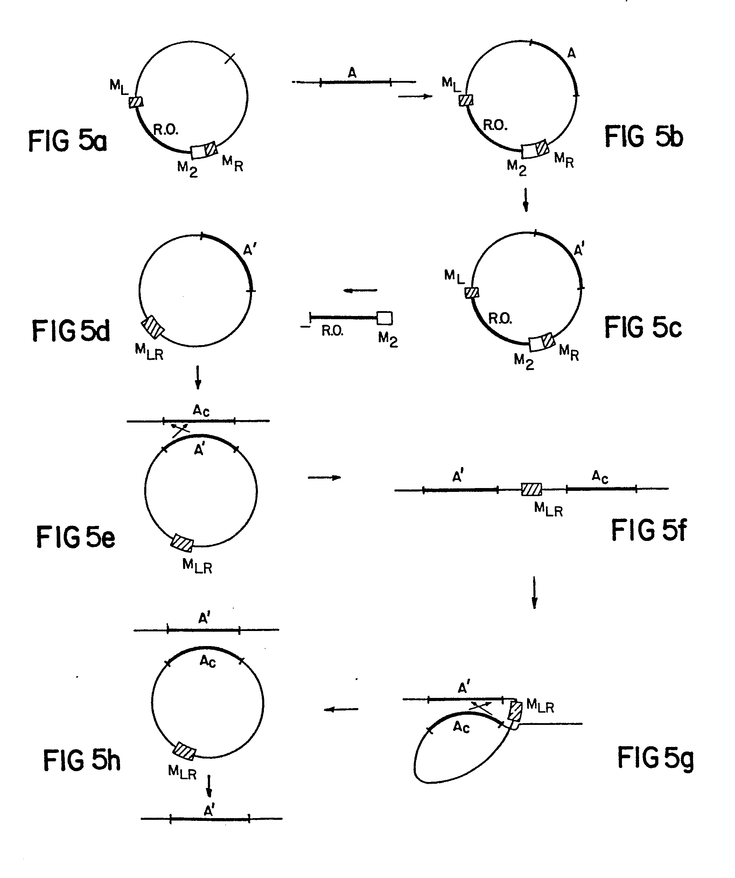

Finally, from clones that are tetracycline resistant, it is possible to select for sub-clones that have undergone excision of the tetracycline gene and therefore also have undergone the desired excision of the homologous portion of the original chromosome ($A_c$ in Fig. 5) or the undesired excision of, e.g., the homologous segment A' originating from pKB843. Selecting against $tet^R$ may be performed, for example by the general method disclosed in Bochner et al. (1980) J. Bacteriology 143:926-933.

Having selected for a population highly enriched in cells which have undergone the desired excision, it is much easier then to identify such cells, for example by screening or selecting for the phenotypical trait resulting from that excision, or by simple screening procedures that indicate the correct DNA structure such as Southern blot analysis after restriction endonuclease digestion of whole cell DNA.

Fig. 5 illustrates generally the use of a vector based on a precursor such as pKB843 that includes a first marker gene divided into two halves ($M_L$ and $M_R$, respectively in Fig. 5) by the excisable replication-determining DNA segment (R.O in Fig. 5). The replication-determining segment also contains a second marker gene, $M_2$. In other respects, the labels in Fig. 5 are as described above for Fig. 1. The steps involved are the same, except that selection procedures described above for pKB843 are enabled, e.g., at step 5f to determine the absence of $M_2$ and the presence of $M_{LR}$; at step 5h, $M_{LR}$ is selected against.

Other arrangements are envisaged within the broad scope of the invention. For example, the method can be used to insert an engineered gene coding for production of any desired protein.

1

0174096

CLAIMS

1. A vector characterised in comprising:
a replication-specific sequence, having a first cleavage site recognised by a restriction endonuclease adjacent one end of said replication specific sequence, and a second cleavage site recognised by a restriction endonuclease adjacent the other end of said replication specific-sequence,
said first and said second sites being the only sites on said vector recognised by a restriction endonuclease which recognises either said first or said second cleavage site;
an engineered DNA sequence to be integrated into the chromosome of a selected bacterial cell; and
means allowing detection of bacterial cells that carry said engineered sequence; and further characterised in that said vector is capable of stable maintenance and autonomous replication of said engineered sequence in a bacterial cell; and in that, upon excision of said replication-specific sequence, said engineered sequence is effective to integrate into the chromosome of a bacterial cell of the same type as the cell in which said vector can be stably maintained, the engineered sequence so integrated then being replicated as part of said chromosome.

2. A vector according to Claim 1, further characterised in that said restriction endonuclease which recognises said first cleavage site is the same as said restriction endonuclease which recognises said second cleavage site.

3. A vector according to Claims 1 or 2, further characterised in that said detection means comprises a gene conferring resistance to an antibiotic.

4. A vector according to any preceding claim, further characterised in that said bacterial cell in which the vector is capable of replication is E. coli.

5. A vector according to any preceding claim, further characterised in that said replication-specific sequence is the origin of replication of pBR322.

6. A vector according to any preceding claim, further characterised in that said engineered sequence comprises a first flanking DNA sequence at one of its ends and a second flanking DNA sequence at its opposite end, and in that said bacterial chromosome comprises a DNA sequence to be replaced which comprises a sequence at one of its ends homologous to said first flanking sequence and a sequence at its opposite end homologous to said second flanking sequence.

7. A vector according to Claim 6, further characterised in that said chromosomal sequence to be replaced comprises a gene coding for expression of a protein, and said engineered sequence comprises a gene engineered from said chromosomal gene.

8. A vector according to Claim 6, further characterised in that said chromosomal sequence to be replaced comprises a gene flanked on one of its sides by said first homologous chromosomal sequence and flanked on its opposite side by said second homologous chromosomal sequence, and in that said engineered DNA consists essentially of said first and said second flanking sequences.

9. A vector according to any preceding claim, further characterised in that said engineered sequence comprises a structural gene coding for a protein whose production is desired.

10. A vector according to any preceding claim, further characterised in that said vector comprises a marker coding for a selectable phenotypical trait between said first and said second restriction cleavage sites to mark the presence of said replication-specific sequence.

11. A vector according to any of Claims 1 to 9, further characterised in that said vector comprises a marker gene coding for a selectable phenotypical trait, said gene being divided into two non-functional fragments by the portion of said vector between said first and said second restriction cleavage sites, and said gene being expressed when said portion is excised.

12. A vector according to Claims 10 or 11, further characterised in that said phenotypical trait can be selected for or against.

13. A vector according to Claim 12, further characterised in that said phenotypical trait is tetracycline resistance.

14. A vector according to Claims 10 or 11, further characterised in that said phenotypical trait is ampicillin resistance.

15. A vector according to any preceding claim, further characterised in that said vector is a derivative of pKB701, ATCC accession no. 39772 or of pKB843, ATCC accession no. 53181.

16. A method by which artificially engineered DNA can be introduced into the chromosome of a bacterial cell to replace a segment of chromosomal DNA, characterised in that it comprises the steps of:

(a) maintaining said engineered DNA in a first bacterial cell on a vector according to any preceding claim;

(b) exposing said vector to a said restriction endonuclease, and removing a DNA segment comprising said replication-specific sequence;

(c) recircularizing the remaining DNA;

(d) transforming a second bacterial cell of the same type as said first cell with said recircularized remaining DNA so that said engineered DNA is incorporated by recombination into the chromosome of said cell and thereby replaces said segment of chromosomal DNA; and

(e)  identifying descendants of said transformed cells in which the engineered DNA is integrated into the chromosome.

17.  A method by which artificially engineered DNA can be introduced into the chromosome of a bacterial cell to replace a segment of chromosomal DNA, characterised in that it comprises the steps of

(a)  maintaining said engineered DNA in a first bacterial cell on a vector comprising:  the said engineered DNA, a replication-specific sequence, having a first cleavage site recognised by a restriction endonuclease adjacent one end of said replication-specific sequence, and a second cleavage site recognised by a restriction endonuclease adjacent the other end of said replication specific-sequence, said first and said second sites being the only sites on said vector recognised by a restriction endonuclease which recognises either said first or said second cleavage site, and means allowing detection of bacterial cells that carry said engineered sequence; said vector being capable of stable maintenance and autonomous replication of said engineered sequence in said first bacterial cell;

(b)  exposing said vector to a said restriction endonuclease, and removing a DNA segment comprising said replication-specific sequence;

(c)  recircularising the remaining DNA;

(d)  transforming a second bacterial cell of the same type as said first cell with said recircularized remaining DNA so that said engineered DNA is incorporated by recombination into the chromosome of said cell and thereby replaces said segment of chromosomal DNA; and

(e)  identifying descendants of said transformed cells in which the engineered DNA is integrated into the chromosome.

18. A method according to Claim 16 or Claim 17, further characterised in that at least some of the artificial engineering of said engineered DNA takes place in said first bacterial cell.

19. A method for the microbiological production of a desired protein characterised in that the method comprises the steps of performing a method according to any of Claims 16, 17 or 18, said engineered sequence on said vector comprising a structural gene coding for said protein; and culturing said second bacterial cell or its progeny.

20. A method according to Claims 16 or 17, for specifically deleting a chromosomally located gene from a bacterial cell, further characterised in that said engineered DNA is formed by ligating together in the correct orientation two discrete DNA sequences, one of which is naturally located immediately 5' to the gene to be deleted, and the other ordinarily located 3' to the said gene to form a hybrid DNA molecule; and in that the step of transforming said bacterial cell with said recircularized remaining DNA, is effective to incorporate said hybrid DNA molecule by recombination into the chromosome of said cell and thereby to replace said chromosomally located gene.

21. A method for deleting a specific chromosomally located gene from a bacterial cell, said method being characterised in comprising the steps of:

(a) forming an engineered DNA sequence by ligating together in the correct orientation two discrete DNA sequences, one of which is naturally located immediately 5' to the gene to be deleted, and the other ordinarily located 3' to the said gene to form a hybrid DNA molecule;

(b) inserting said hybrid DNA molecule into a vector from which a DNA sequence essential for replication can be conveniently excised;

(c) excising said replication sequence from the vector and recircularizing the remaining DNA;

(d) transforming a said bacterial cell with said recircularized remaining DNA, so that said hybrid DNA molecule is incorporated by recombination into the chromosome of said cell and thereby replaces said chromosomally located gene; and

(e) identifying descendants of the said cells having said hybrid DNA molecule integrated into said cell's chromosome in place of said gene.

22. A precursor to a vector according to any of Claims 1 to 15 characterised in comprising:

a said replication-specific sequence, having

a first cleavage site recognised by a restriction endonuclease adjacent one end of said replication specific sequence, and

a second cleavage site recognised by a restriction endonuclease adjacent the other end of said replication specific sequence,

said first and said second sites being the only sites on said vector recognised by a restriction endonuclease which recognises either said first or said second sites; and

at least one cleavage site positioned outside said replication specific sequence and recognised by a third restriction endonuclease that does not recognise either said first or said second site.

23. A vector according to Claim 22, further characterised in that said vector is pKB701, ATCC accession no. 39772, pKB843, ATCC accession no. 53181, or derivatives thereof.

0174096

FIG Ia

FIG Ib

FIG Id

FIG Ic

FIG Ie

FIG If

FIG Ih

FIG Ig

FIG 2

FIG 3

FIG 4

Piece A

EcoRI

Bam HI

5' portion
of tet$^R$

ori

pKB843

Piece B
(ori & amp$^R$)

amp$^R$

Piece C

3' portion
of tet$^R$

Bam HI

FIG 5a

FIG 5b

FIG 5c

FIG 5d

FIG 5e

FIG 5f

FIG 5g

FIG 5h